(19) Europäisches Patentamt
European Patent Office
Office européen des brevets

(11) Publication number: **0 146 626**
**A1**

(12) # EUROPEAN PATENT APPLICATION

published in accordance with Art. 158(3) EPC

(21) Application number: **83901730.8**

(22) Date of filing: **02.06.83**

Data of the international application taken as a basis

(86) International application number:
**PCT/JP83/00181**

(87) International publication number
**WO84/04747 (06.12.84 84/28)**

(51) Int. Cl.[4]: **C 07 D 501/04**
C 07 D 501/18, C 07 D 501/36
A 61 K 31/545

(43) Date of publication of application:
**03.07.85 Bulletin 85/27**

(84) Designated Contracting States:
**CH DE FR GB LI SE**

(71) Applicant: **Kyoto Pharmaceutical Industries, Ltd**
**38, Nishinokyo Tsukinowa-cho Nakakyo-ku**
**Kyoto-shi Kyoto 604(JP)**

(72) Inventor: **KAKEYA, Nobuharu**
**10-16, Takadai 3-chome**
**Nagaokakyo-shi Kyoto 617(JP)**

(72) Inventor: **KITAO, Kazuhiko**
**12, Sandan Osa-cho Matugasaki Sakyo-ku**
**Kyoto-shi Kyoto 606(JP)**

(74) Representative: **von Kreisler, Alek, Dipl.-Chem. et al,**
**Deichmannhaus am Hauptbahnhof**
**D-5000 Köln 1(DE)**

(54) **CEPHEM COMPOUNDS.**

(57) Cephem compounds represented by general formula (I), wherein $R^1$ represents a hydrogen atom or an amino group-protecting group and $R_2$ represents formula (II) or (III), wherein $R^3$ represents a lower alkyl group and $R^4$ represents a lower alkyl group or an alkoxy group, and salts thereof. These compounds are useful as starting materials for synthesizing cephalosporin derivatives for oral administration.

EP 0 146 626 A1

## Specification

## Cephem Compounds

## Technical Field

This invention relates to a novel cephem compounds useful as the materials for preparing cephalosporin derivatives for oral administration and a method of preparing thereof.

## Disclosure of the Invention

This invention relates to cephem compounds, inclusive salts thereof, representable by the general formula;

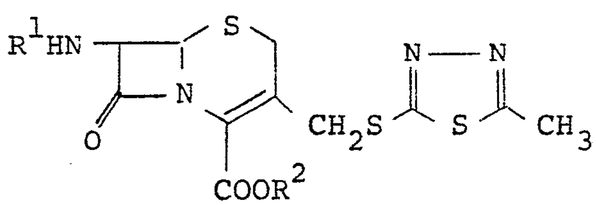

(I)

(wherein $R^1$ stands for hydrogen atom or a protective group of amino group, $R^2$ stands for a group representable by the formula;

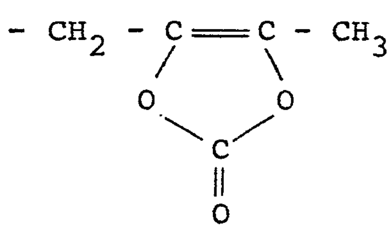

or

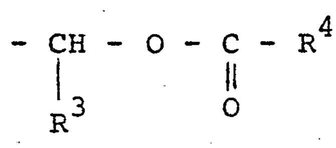

where $R^3$ stands for a lower alkyl group and $R^4$ stands for a lower alkyl group or alkoxy group.) and a method of preparing thereof.

Cephalosporins are possessed of a remarkably strong antibiotic action, but most of them are hardly absorbed from digestive tract when administered orally. Almost all of cephalosporins are therefore administered by non-oral route such as intramuscularly.

The present inventors have conducted extensive study on creation of such cephalosporin derivatives as can be absorbed into blood even by oral administration to find out that the cephalosporin derivatives prepared by acylation of the cephem compounds (I) above can be readily absorbed into blood even by oral administration. The present invention is thus to provide the compounds useful as, among others, the materials for preparing cephalosporin derivatives for oral administration.

Throughout this specification, lower alkyl groups are straight-chained or branched, and they are in general of $C_{1-4}$, more specifically, methyl, ethyl, n-propyl, iso-propyl, n-butyl, iso-butyl, t-butyl, etc.

Lower alkoxy groups are straight-chained or branched ones, and they are in general of $C_{1-4}$, more specifically, methoxy, ethoxy, n-propoxy, iso-propoxy, n-butoxy, iso-butoxy, t-butoxy, etc.

As the protective groups of amino group, there may be mentioned per se known ones such as benzylcarbonyl, formyl, t-butoxycarbonyl, D-5-amino-5-carboxyvaleryl, trityl, phthalimido, 1-methyl-2-ethoxycarbonylvinyl, etc.

The cephem compounds (I) of this invention can be prepared by, for example, allowing a compound representable by the general formula,

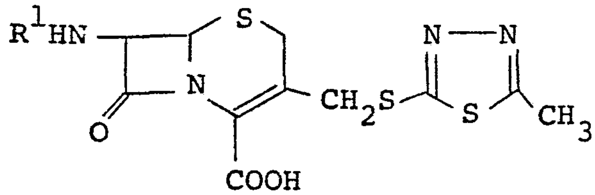

(II)

(wherein $R^1$ is of the same meaning as defined above) to react with a compound representable by the general formula;

$$X - R^2 \qquad \text{(III)}$$

[wherein $R^2$ is of the same meaning as defined above, and X stands for "a group reactive with carboxyl group (its reactive group)"].

As the "group reactive with carboxyl group (its reactive group)" shown by X in the general formula (III), there may be exemplified halogen (bromine, chlorine, iodine, etc.), alkylsulfonyloxy (methanesulfonyloxy, etc.), arylsulfonyloxy (p-toluenesulfonyloxy, etc.), hydroxyl group, etc.

The compound (II) is subjected to reaction preferably as, in general, its reactive derivative (for example, an alkaline metal salt such as sodium salt, potassium salt, etc., an alkaline earth metal salt such as calcium salt, etc., triethylamine salt, pyridine salt, etc.). Incidentally, when a compound (III) where X is hydroxyl group is employed, it is preferable to use the compound (II) as a free acid, and, in that case, use of suitable condensing agent is preferable. As the condensing agent, there may, for example, be employed such dehydrating agent as N,N'-disubstituted carbodiimides (e.g. N,N'-dicyclohexylcarbodiimide) and azolide compounds (e.g. N,N'-carbonyldiimidazole, N,N'-thionyldiimidazole). When any of those condensing agents is used, the reaction is considered to proceed via a reactive derivative of carboxylic acid.

The reaction of a compound (II) with a compound (III) is preferably conducted under cooling so as to avoid by-production of $\Delta^2$-isomer, and the reaction may be allowed to proceed in a solvent inert to the reaction (for example, dimethylformamide,

dimethylacetamide, hexamethyl phosphoro triamide, acetone, acetonitrile, etc.).

In the reaction of this invention, $R^1$ of the general formula (II) may be an amino-protecting group, and, in this case, the reaction between a compound (II) and a compound (III) gives a cephem compound (I) whose amino group at 7-position is protected. This protecting group can be removed by a per se known method.

As the method of removing such protecting group, when removal of benzylcarbonyl, 2-thienylacetyl, 2-furylacetyl, D-5-amino-5-carboxyvaleryl, etc. is intended, decomposition with methanol through iminochlorination with phosphorus pentachloride is resorted to for example; when removal of formyl, t-butoxycarbonyl, 1-methyl-2-ethoxycarbonyl vinyl, trityl, etc. is intended, treatment with an acid (e.g. formic acid, trifluoroacetic acid or hydrochloric acid) is mentioned, for example; and when removal of phthalimido is intended, Ing-Manske's method employing hydrazine may be counted, for example.

The cephem compounds (I) can be separated from the reaction mixture and purified by per se known means. When $R^1$ in the general formula (I) is hydrogen, the cephem compound is isolated as an acid-addition salt which is in general of higher stability. As an acid to form such an acid-addition salt, there may be exemplified mineral acids such as hydrochloric acid, sulfuric acid, phosphoric acid, nitric acid, etc., or (non-toxic) organic acids such as oxalic acid, fumaric acid, maleic acid, citric acid, tartaric acid, methanesulfonic acid, p-toluene sulfonic acid, etc.

Incidentally, the cephem compounds (I) thus produced can be fed to the subsequent reaction step for producing, among others, a cephalosporin derivative, without isolation from the reaction mixture.

Thus-produced cephem compounds (I) can be used as materials for synthesizing cephalosporin derivatives administrable per os by acylating the amino group at 7-position thereof.

Production of the orally administrable cephalosporin derivatives from the cephem compounds (I) of this invention can be conducted by a per se known acylation method.

Example 1

Synthesis of (5-methyl-1,3-dioxol-2-on-4-ylmethyl) 7-amino-3-[(5-methyl-1,3,4-thiadiazol-2-yl)thiomethyl]-3-cephem-4-carboxylate (Process 1)

To 250 ml of a mixture solution of dimethyl sulfoxide and dimethyl formamide was added 12.4 g of potassium 7-amino-3-[(5-methyl-1,3,4-thiadiazol-2-yl)thiomethyl]-3-cephem-4-carboxylate. To the mixture was added at 0°C 7.51 g of 4-bromomethyl-5-methyl-1,3-dioxol-2-one, which was stirred at the same temperature for 30 minutes. The reaction solution was dissolved in ethyl acetate, which was washed with water and saline successively, then dried on anhydrous sodium sulfate, followed by removal of the solvent by evaporation. The residue was purified by means of a silica-gel column (eluent, benzene:ethyl acetate, 7:3), then crystallized from petroleum ether to give 6.3 g of the above-titled compound.

I.R. (Nujol , $cm^{-1}$)

3400, 3350, 1810, 1780, 1730

N.M.R. ($CDCl_3$, δ value)

1.83 (s, 2H, $-NH_2$)

2.22 (s, 3H, dioxole $-CH_3$)

2.71 (s, 3H, thiadiazole $-CH_3$)

3.33, 3.86 (d, d, 2H, J=18Hz, 2-position $-CH_2-$)

3.91, 4.88 (d, d, 2H, J=14Hz, 3-position $-CH_2S-$)

4.78 ~ 4.95 (m, 2H, 6-position $-\overset{|}{\underset{|}{C}}H$, 7-position $-\overset{|}{\underset{|}{C}}H$)

4.90, 5.19 (d, d, 2H, J=15Hz, dioxole $-CH_2-$)

(Process 2)

In 10 ml of a mixture solution of dimethylsulfoxide-dimethylformamide (1 : 1) was dissolved 380 mg of potassium salt of 7-amino-3-[(5-methyl-1,3,4-thiadiazol-2-yl)thiomethyl]-3-cephem-4-carboxylic acid. To the solution was added at -10°C 340 mg of (5-methyl-1,3-dioxol-2-on-4-ylmethyl) methanesulfonate dissolved in 1 ml of dimethylformamide, and the reaction was allowed to proceed for 30 minutes. To the reaction mixture was then added water, which was subjected to extraction with ethyl acetate. The ethyl acetate layer was washed with a saturated aqueous solution of sodium hydrogen carbonate then with saturated saline, followed by drying on anhydrous sodium sulfate. The solution was concentrated to dryness under reduced pressure, to which was added petroleum ether to cause solidification, followed by drying to give 170 mg of the above-titled compound.

(Process 3)

In 150 ml of dimethylformamide was dissolved 6.2 g of potassium salt of 7-formamido-3-[(5-methyl-1,3,4-thiadiazol-2-yl)

BAD ORIGINAL

thiomethyl]-3-cephem-4-carboxylic acid. To the solution was added at -5°C 3.8 g of 4-bromomethyl-5-methyl-1,3-dioxol-2-one, which was stirred for 30 minutes. To the reaction mixture was added water, which was subjected to extraction with ethyl acetate. The extract was washed with a saturated aqueous solution of sodium hydrogen carbonate, water and aqueous saline successively, followed by drying on anhydrous sodium sulfate. The dried product was concentrated and crystallized from isopropylether to give 4.4 g of (5-methyl-1,3-dioxol-2-on-4-ylmethyl) 7-formamido-3-[(5-methyl-1,3,4-thiadiazol-2-yl)thiomethyl]-3-cephem-4-carboxylate.

I.R. (Nujol, $cm^{-1}$)

1810, 1730, 1650

N.M.R. ($CDC\ell_3$, δ value)

2.30 (s, 3H, dioxole $-CH_3$)

2.73 (s, 3H, thiadiazole $-CH_3$)

3.35, 3.88 (d, d, 2H, J=18Hz, 2-position $-CH_2-$)

3.91, 4.90 (d, d, 2H, J=14Hz, 3-position $-CH_2S-$)

5.13 (d, 1H, 6-position $-\overset{|}{\underset{|}{C}}H$)

5.74 (d, d, 1H, 7-position $-\overset{|}{\underset{|}{C}}H$)

4.91, 5.19 (d, d, 2H, J=15Hz, dioxole $-CH_2-$)

8.11 (s, 1H, -CHO)

9.60 (d, 1H, -CONH-)

In 50 ml of methanol was dissolved 6 g of thus obtained compound. To the solution was added at 0°C 5 ml of concentrated hydrochloric acid, and the mixture was stirred for 6 hours.

BAD ORIGINAL

The reaction mixture was subjected to extraction with ethyl acetate. The extract was washed with a dilute aqueous solution of sodium hydrogen carbonate, water and saline solution successively, dried on anhydrous sodium sulfate, followed by removal of the solvent by evaporation to give 4.2 g of the above-titled compound.

Example 2-4

By similar methods to "Process 1" of Example 1, the following compounds were prepared:

1-Acetoxyethyl 7-amino-3-[(5-methyl-1,3,4-thiadiazol-2-yl) thiomethyl]-3-cephem-4-carboxylate

I.R. (Nujol, $cm^{-1}$)

3450, 3350, 1780, 1760

N.M.R. ($CDCl_3$, δ value)

1.57 (d, 3H, J=6Hz, $-\overset{|}{C}HCH_3$)

1.61 (s, 2H, $NH_2$)

2.10 (s, 3H, $-COCH_3$)

2.71 (s, 3H, thiadiazole $-CH_3$)

3.68 (s, 2H, 2-position $-CH_2-$)

4.16, 4.65 (d, d, 2H, J=13Hz, 3-position $-CH_2S-$)

4.69, 4.90 (m, 2H, 6-position $-\underset{|}{\overset{|}{C}}H$, 7-position $-\underset{|}{\overset{|}{C}}H$)

6.98, 7.08 (q, q, 1H, J=6Hz, $-\overset{|}{C}H-CH_3$)

1-Isobutyryloxyethyl 7-amino-3-[(5-methyl-1,3,4-thiadiazol-2-yl)thiomethyl]-3-cephem-4-carboxylate

BAD ORIGINAL

I.R. (Nujol, $cm^{-1}$)

3350, 1780, 1735

N.M.R. ($CDCl_3$, δ value)

1.18 (d, 6H, J=7Hz, $-CH(CH_3)_2$)

1.55, 1.58 (d, d, 3H, J=6Hz, $-CHCH_3$)

2.10 (br, s, 2H, $-NH_2$)

2.3 ~ 3.0 (m, 1H, $-CH(CH_3)_2$)

3.71 (m, 2H, 2-position $-CH_2-$)

4.13, 4.57 (d, d, 2H, J=14Hz, 3-position $-CH_2S-$)

4.69 ~ 4.92 (m, 2H, 6-position $-\overset{|}{\underset{|}{C}}H$, 7-position $-\overset{|}{\underset{|}{C}}H$)

6.95 (m, 1H, $-CHCH_3$)

1-Pivaloyloxyethyl 7-amino-3-[(5-methyl-1,3,4-thiadiazol-2-yl) thiomethyl]-3-cephem-4-carboxylate

I.R. (Nujol, $cm^{-1}$)

3400, 3340, 1780

N.M.R. ($CDCl_3$, δ value)

1.21 (s, 9H, $-C(CH_3)_3$)

1.54, 1.57 (d, d, 3H, J=6Hz, $-CHCH_3$)

1.93 (br, 2H, $NH_2$)

2.71 (s, 3H, thiadiazole $-CH_3$)

3.67 (br, 2H, 2-position $-CH_2-$)

4.11, 4.65 (d, d, 2H, J=14Hz, 3-position $-CH_2S-$)

4.71 ~ 4.91 (m, 2H, 6-position $-\overset{|}{\underset{|}{C}}H$, 7-position $-\overset{|}{\underset{|}{C}}H$)

7.0 (m, 1H, $-CHCH_3$)

Example 6

Synthesis of 1'-ethoxycarbonyloxyethyl 7-amino-3-[(5-methyl-1,3,4-thiadiazol-2-yl)thiomethyl]-3-cephem-4-carboxylate

In 200 ml of dimethylsulfoxide were dissolved 10 g of 7-amino-3-[(5-methyl-1,3,4-thiadiazol-2-yl)thiomethyl]-3-cephem-4-carboxylic acid and 2.8 g of potassium acetate. To the solution was added 8.5 g of α-iododiethylcarbonate, and the mixture was stirred at room temperature for two hours. The reaction solution was dissolved in ethyl acetate, washed with water and aqueous saline successively, dried on anhydrous sodium sulfate, followed by removal of the solvent by evaporation. The residue was purified by means of a silica-gel column to give 5.1 g of the above-titled compound.

I.R. (Nujol, $cm^{-1}$)

3400, 1775

N.M.R. ($CDCl_3$, δ value)

1.32 (d, 3H, J-7Hz, $-CH_2CH_3$)

1.60, 1.62 (d, d, 3H, J=5Hz $-CHCH_3$)

1.85 (br, s, 2H, $-NH_2$)

2.72 (s, 3H, thiadiazole $-CH_3$)

3.69 (m, 2H, 2-position $-CH_2-$)

4.20, 4.62 (d, d, 2H, 3-position $-CH_2S-$)

4.25 (q, 2H, J=7Hz, $-CH_2CH_3$)

4.73, 4.94 (m, 2H, 6-position $-\overset{|}{\underset{|}{C}}H$, 7-position $-\overset{|}{\underset{|}{C}}H$ )

6.90, 7.00 (q, q, 1H, J=5Hz, $-CHCH_3$)

BAD ORIGINAL

0146626

Example 7

Preparation of (5-methyl-1,3-dioxol-2-on-4-ylmethyl) 7-amino-3-[(5-methyl-1,3,4-thiadiazol-2-yl)thiomethyl]-3-cephem-4-carboxylate:

(1) In a mixture of 90 ml of methylene chloride and 10 ml of dimethylformamide were dissolved 1.85 g of 7-formamido-3-[(5-methyl-1,3,4-thiadiazol-2-yl)thiomethyl]-3-cephem-4-carboxylic acid and 0.7 g of 4-hydroxymethyl-5-methyl-1,3-dioxol-2-one. To the solution was added at 0°C 1.1 g of dicyclohexylcarbodiimide, and the mixture was allowed to react at 0-5°C for two hours. After the reaction, the resulting precipitates were removed by filtration. The filtrate was washed with a saturated aqueous solution of sodium hydrogen carbonate and saturated aqueous saline, successively, followed by drying on anhydrous sodium sulfate. This solution was concentrated under reduced pressure. To the concentrate was added ethyl acetate, and the resulting small amount of precipitates was again removed by filtration. The filtrate was concentrated, and the residue was subjected to chromatography using 50 g of silica-gel and benzene-ethyl acetate (1:3) as a eluent to give 0.7 g of (5-methyl-1,3-dioxol-2-on-4-ylmethyl)-7-foramido-3-[(5-methyl-1,3,4-thiadiazol-2-yl)thiomethyl]-3-cephem-4-carboxylate.

I.R. (Nujol, $cm^{-1}$)

1810, 1730, 1650

N.M.R. ($CDCl_3$, $\delta$ value)

2.30 (s, 3H, dioxole $-CH_3$)

2.73 (s, 3H, thiadiazole $-CH_3$)

3.35, 3.88 (d, d, 2H, J=18Hz, 2-position $-CH_2-$)

3.91, 4.90 (d, d, 2H, J=14Hz, 3-position $-CH_2S-$)

5.13 (d, 1H, 6-position -CH)

5.74 (d, d, 1H, 7-position -CH )

4.91, 5.19 (d, d, 2H, J=15Hz, dioxole $-CH_2-$)

8.11 (s, 1H, -CHO)

9.60 (d, 1H, -CONH-)

(2) In 7 ml of methanol was dissolved 0.7 g of the compound obtained in (1) above. To the solution was added 1 ml of concentrated hydrochloric acid at 0°C, and the mixture was stirred at room temperature for six hours. The reaction solution was subjected to extraction with ethyl acetate, and the extract was washed with dilute aqueous solution of sodium hydrogen carbonate, water and saturated aqueous saline, successively, followed by drying on anhydrous sodium sulfate. The solution thus obtained was concentrated under reduced pressure, to which was added isopropyl ether to cause solidification, followed by drying to give 0.53 g of the above-titled compound.

I.R. (Nujol, $cm^{-1}$)

3400, 3350, 1810, 1780, 1730

N.M.R. ($CDCl_3$, δ value)

1.83 (br, s, 2H, $-NH_2$)

2.22 (s, 3H, dioxole $-CH_3$)

2.71 (s, 3H, thiadiazole $-CH_3$)

3.33, 3.86 (d, d, 2H, J=18Hz, 2-position $-CH_2$)

3.91, 4.88 (d, d, 2H, J=14Hz, 3-position $-CH_2S-$)

4.78 ~ 4.95 (m, 2H, 6-position -CH, 7-position -CH )

4.90, 4.90, 5.19 (d, d, 2H, J=15Hz, dioxole $-CH_2-$)

What is claimed is:

1. A cephem compound representable by the general formula;

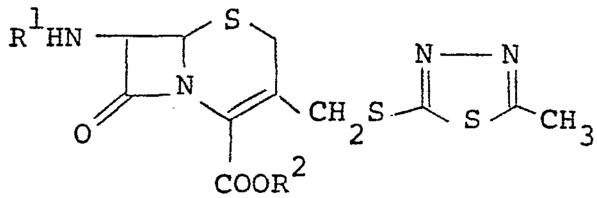

(wherein $R^1$ stands for hydrogen atom or a protective group of amino group, and $R^2$ stands for a group representable by the formula;

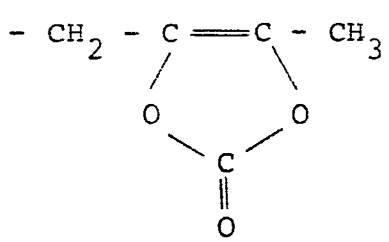

or

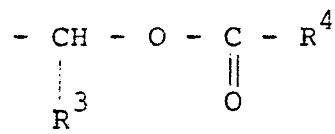

wherein $R^3$ stands for a lower alkyl group and $R^4$ stands for a lower alkyl group or a lower alkoxy group)
or a salt thereof.

2. The cephem compound claimed in Claim 1, wherein $R^2$ is a group representable by the formula;

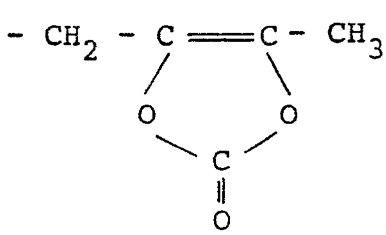

3. The cephem compound claimed in Claim 1, wherein $R^2$ is representable by the formula;

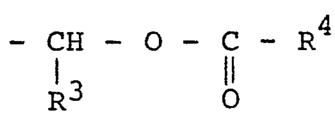

(wherein $R^3$ stands for a lower alkyl group and $R^4$ stands for a lower alkyl group or a lower alkoxy group).

4. The cephem compound claimed in Claim 1, wherein $R^3$ and $R^4$ respectively stand for methyl group.

5. The cephem compound claimed in Claim 1, wherein $R^3$ stands for methyl and $R^4$ stands for ethoxy.

6. The cephem compound claimed in Claims 1-5, wherein $R^1$ stands for hydrogen atom.

7. The cephem compound claimed in Claims 1-5, wherein $R^1$ stands for a protective group of amino group.

8. A method of preparing a cephem compound as claimed in Claim 1, which is characterised by allowing a compound representable by the general formula;

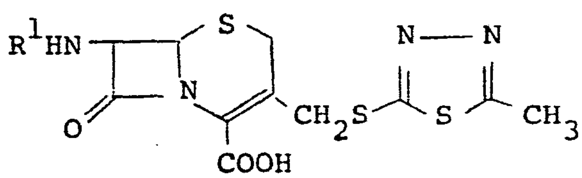

(wherein $R^1$ stands for hydrogen atom or a protective group of amino group) to react with a compound representable by the general formula;

$$X - R^2$$

(wherein $R^2$ stands for a group representable by the formula;

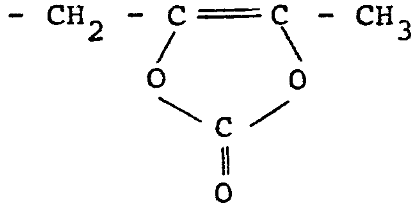

or

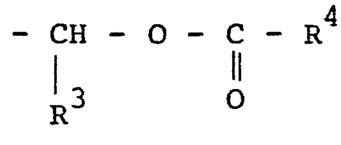

where $R^3$ stands for a lower alkyl group and $R^4$ stands for a lower alkyl group or a lower alkoxy group).

0146626

# INTERNATIONAL SEARCH REPORT

International Application No. PCT/JP83/00181

**I. CLASSIFICATION OF SUBJECT MATTER** (if several classification symbols apply, indicate all) [3]

According to International Patent Classification (IPC) or to both National Classification and IPC

Int. Cl.$^3$ C07D 501/04, 501/18, 501/36, A61K 31/545

**II. FIELDS SEARCHED**

Minimum Documentation Searched [4]

| Classification System | Classification Symbols |
|---|---|
| I P C | C07D 501/04, 501/18, 501/36, A61K 31/545 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No [18] |
|---|---|---|
| X | JP,A, 51-16687 (Eli Lilly and Co.) 10. February. 1976 (10.02.76) | 1 |
| X | JP,A, 56-86188 (Fujisawa Pharmaceutical Co., Ltd.) 13. July. 1981 (13.07.81) | 1 |
| X | JP,A, 56-128786 (Roussel Hucraff) 8. October. 1981 (08.10.81) | 1 |
| X | JP,A, 57-59894 (Sankyo Co., Ltd.) 10. April. 1982 (10.04.82) | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| August 22, 1983 (22.08.83) | September 5, 1983 (05.09.83) |
| **International Searching Authority** [1] | **Signature of Authorized Officer** [20] |
| Japanese Patent Office | |